# EUROPÄISCHE PATENTANMELDUNG

(11) **EP 0 763 721 A1**
(43) Veröffentlichungstag der Anmeldung: **19.03.1997**
(21) Anmeldenummer: 96114358.3
(22) Anmeldetag: 07.09.1996
(51) Int. Cl.: G01F 11/26

(54) **Dosier-Verschluss für Flüssigkeiten**

(30) Priorität: 13.09.1995 DE 29514691 U
(71) Anmelder: Friedrich Sanner GmbH & Co. KG Spritzgusswerk, D-64625 Bensheim 3-Auerbach (DE)
(72) Erfinder: Oschmann, Markus, Dipl.-Ing., 64653 Lorsch (DE)
(74) Vertreter: Helber, Friedrich G., Dipl.-Ing.

(57) **Zusammenfassung**

Dosier-Verschluß (10) für Flüssigkeits-Behälter mit einer im Ausguß des Behälters angeordneten Dosierkammer in Form eines in einem Behälter innenseitig durch eine Stirnwand geschlossenen und ausgußseitig offenen, am Behälterausguß abdichtenden Hohlstopfens (12). In der Stirnwand des Hohlstopfens ist eine federnd in die Schließstellung vorgespannte Ventileinrichtung vorgesehen, die zum Übertritt einer vorgegebenen Menge von Flüssigkeit aus dem Behälterinnern in die Dosierkammer geöffnet werden kann, wobei der Behälter und zugleich die offene Stirnseite des Hohlstopfens (12) durch eine abnehmbare Verschlußkappe (14) verschlossen sind. Der dem Durchlaß im Hohlstopfen (12) gegenüberliegende Deckwandbereich (38) ist elastisch ins Hohlstopfeninnere verformbar ausgebildet, und zwischen der der Dosierkammer zugewandten Innenseite des elastisch verformbaren Deckwandbereichs und der Ventileinrichtung ist ein die Dosierkammer durchsetzender Stößel (34) angeordnet, dessen Länge so bemessen ist, daß er bei einer Verformung des Deckwandbereichs in Richtung des Behälterinnern die Ventileinrichtung aus ihrer Schließstellung in die Öffnungsstellung verstellt.

Die Ventileinrichtung wird von freigschnittenen zungenartigen Wandbereichen (28) in der als durchgewölbte, elastisch in Richtung des Behälterinnern verformbare Membran ausgebildeten Stirnwand des Hohlstopfens (12) gebildet.

## Beschreibung

Die Erfindung betrifft ein Dosier-Verschluß für zur Aufnahme von Flüssigkeiten bestimmte Behälter mit einer im Ausguß des die Flüssigkeit aufnehmende Behälters angeordneten Dosierkammer in Form eines in einem behälterinnenseitig durch eine Stirnwand geschlossenen und ausgußseitig offenen, am Behälterausguß abdichtenden Hohlstopfens, in dessen Stirnwand eine federnd in die Schließstellung vorgespannte Ventileinrichtung vorgesehen ist, die zum Übertritt einer vorgegebenen Menge von Flüssigkeit aus dem Behälterinnern in die Dosierkammer geöffnet werden kann, wobei der Behälter und zugleich die offene Stirnseite des Hohlstopfens durch eine abnehmbare Verschlußkappe verschlossen sind, deren dem Durchlaß im Hohlstopfen gegenüberliegender Deckwandbereich elastisch in Richtung ins Hohlstopfeninnere verformbar ausgebildet ist und zwischen der der Dosierkammer zugewandten Innenseite des elastisch verformbaren Deckwandbereichs und der Ventileinrichtung ein die Dosierkammer durchsetzender Stößel angeordnet ist, dessen Länge so bemessen ist, daß er bei einer Verformung des Deckwandbereichs in Richtung des Behälterinnern die Ventileinrichtung aus ihrer Schließstellung in die Öffnungsstellung verstellt.

Dosier-Verschlüsse dienen zur Entnahme jeweils einer vorgegebenen Menge einer Flüssigkeit, z.B. eines flüssigen Arzneimittels, aus einem Behälter, beispielsweise einem Glasfläschchen. Gegenüber der unbequemen und auch nicht immer irrtumsfreien Dosierung von Flüssigkeiten aus Tropfflaschen durch Abzählen einer bestimmten Menge von Tropfen wird die dosierte Entnahme der Flüssigkeit erheblich sicherer erreicht und ist auch bequemer. Auch gegenüber der Dosierung von Flüssigkeit mittels einer Pipette hat der in den Behälterhals integrierte Dosier-Verschluß den Vorteil, daß die im Behälter abgefüllte Flüssigkeit auch beim Dosiervorgang selbst und dem Ausgießen der Dosiermenge durch den Verschluß gegen die Umgebungsatmosphäre abgedichtet ist, und es somit nicht zu Verunreinigungen oder Keimbefall kommen kann. Auch ein versehentliches Auslaufen von Flüssigkeit, wenn beispielsweise der Behälter versehentlich umgestoßen wird, ist mit Sicherheit vermieden.

Es ist ein Dosier-Verschluß der eingangs erwähnten Art bekannt (DE-GM 94 07 985), der eine zufriedenstellende Dosier-Genauigkeit und Gebrauchstüchtigkeit aufweist. Soweit der bekannte Verschluß einschließlich der Ventileinrichtung integral im Spritzgußverfahren aus Kunststoff hergestellt wird, erfordert er aber komplizierte Spritzgußformen in dem Bereich, in dem beim Abspritzen die Ventileinrichtung entstehen soll. Beim bekannten Verschluß ist deshalb die Ventileinrichtung bzw. Teile derselben gesondert hergestellt worden, was nachträgliche Montageschritte erfordert. In beiden Fällen wirkt sich dies auf die Herstellungskosten aus.

Der Erfindung liegt die Aufgabe zugrunde, den bekannten Dosier-Verschluß so weiterzubilden, daß er einfacher aufgebaut und somit preisgünstiger herstellbar ist, ohne daß seine Funktion oder Dosiergenauigkeit hierdurch beeinträchtigt wäre.

Ausgehend von dem Dosierverschluß der eingangs erwähnten Art wird diese Aufgabe erfindungsgemäß dadurch gelöst, daß die Stirnwand des Hohlstopfens als in Richtung zum Behälter-Ausguß durchgewölbte und elastisch in Richtung des Behälterinnern verformbaren Membran ausgebildet ist, an welcher das dem Behälterinnern zugewandte Ende des Stößels angreift und daß in der Membran wenigstens ein zungenartiger Wandbereich freigeschnitten ist, dessen freigeschnittene Begrenzungskanten in der Schließstellung den angrenzenden Membran-Wandbereichen ohne Bildung eines merklichen Spalts gegenüberstehen, nach einer Verformung der Membran durch den Stößel in die Öffnungsstellung jedoch nicht mitverformt werden und einen Durchlaß in der Membran für den Übertritt von Flüssigkeit aus dem Behälterinnern in die Dosierkammer freigeben. Die Ventileinrichtung ist somit in der als elastisch verformbaren Membran ausgebildeten behälterinnenseitigen Membran ausgebildet, aus der eine oder mehrere Zungen oder Segmente freigeschnitten sind, die sich bei der Verformung der Membran über den Stößel nicht oder in anderer Weise als die restliche Membran verformen, wodurch im freigeschnittenen Bereich jeweils ein Durchlaß entsteht, durch den die Flüssigkeit - bei auf den Kopf gestelltem Behälter - in die Dosierkammer übertreten kann. Über den Durchlaß bzw. die Durchlässe wird dann auch von der einströmenden Flüssigkeit die in der Dosierkammer enthaltene Luft in den Behälter verdrängt. Der Dosiervorgang erfolgt also - wie beim bekannten Dosier-Verschluß - so, daß bei auf den Kopf gestelltem Behälter der elastisch verformbare Deckwandbereich in Richtung in Behälterinnern gedrückt wird, wobei durch den Stößel die Membran entgegen ihrer Wölbung verformt wird und sich der bzw. die Durchlässe öffnen. Sobald die Dosierkammer gefüllt ist, was daran zu erkennen ist, daß keine Bläschen mehr in der im Filter befindlichen Flüssigkeit hochsteigen, ist der Füllvorgang beendet und der Behälter wird wieder in die Normalstellung gebracht. Dann kann die Behälter-Verschlußkappe abgenommen und diese Dosierkammer entleert werden. D.h. es wird genau die vorgegebene Dosiermenge entnommen.

Der Hohlstopfen einschließlich der die behälterinnenseitige Stirnwand bildenden Membran ist in bevorzugter Ausgestaltung der Erfindung ein integraler Spritzgußteil.

Alternativ kann die die behälterinnenseitige Stirnwand bildende Membran auch gesondert hergestellt und nachträglich abdichtend in die behälterinnere Stirnseite der beidseitig offenen hohlzylindrischen Stopfen-Umfangswandung eingesetzt sein, wobei es sich dann empfiehlt, die Membran mit einem verstärkten Randbereich zu versehen, welcher in eine im behälterinnenseitigen Endbereich der Stopfen-Umfangswandung in deren Innenseite vorgesehene umlaufende Nut eingerastet ist.

Dabei wird dann der Außendurchmesser des verstärkten Randbereichs zweckmäßig mit Übermaß gegenüber dem Durchmesser des Nutgrunds der umlaufenden Nut bemessen, so daß der Randbereich mit Vorspannung in der Nut sitzt und so die einwandfreie Abdichtung gewährleistet ist.

Der Dosiervorgang kann insgesamt beschleunigt werden, wenn in der die behälterseitige Stirnwand bildenden Membran mehrere zungenartige Wandbereiche freigeschnitten sind.

Der zungenartige Wandbereich bzw. die zungenartigen Wandbereiche können dabei in einem gesonderten Arbeitsgang freigestanzt sein. Alternativ können sie auch durch entsprechende Ausformungen der Spritzgußform bei der Herstellung des Hohlstopfens in der Spritzgußform erzeugt sein, wobei beim Spritzvorgang selbst im Bereich der die zungenartigen Wandbereiche begrenzenden Spalt zunächst noch einen dünnen Film aus dem Kunststoffmaterial verbleiben kann, der dann erst bei der erstmaligen Benutzung des Dosier-Verschlusses zerreißt. Bis zur erstmaligen Benutzung ist der Behälterinhalt dann also zusätzlich zur Abdichtung durch die eigentliche Verschlußkappe auch noch durch den Hohlstopfen gegen den Zutritt von Umgebungsatmosphäre abgedichtet.

Die Erfindung ist in der folgenden Beschreibung eines Ausführungsbeispiels in Verbindung mit der Zeichnung näher erläutert, und zwar zeigt:
- Fig. 1: den oberen ausgußseitigen Teil eines zur Aufnahme einer Flüssigkeit bestimmten Fläschchens mit einem im Flaschenhals vorgesehenen, in der erfindungsgemäßen Weise ausgebildeten Dosier-Verschluß im Längsmittelschnitt;
- Fig. 2: eine Ansicht auf die behälterinnenseitige Stirnwand des Teils des Dosier-Verschlusses bildenden Hohlstopfens, gesehen in Richtung des Pfeils 2 in Fig. 1; und
- Fig. 3: eine der Fig. 1 entsprechende Schnittansicht, in welcher der mit dem Dosier-Verschluß versehene Behälter auf den Kopf gestellt ist, wobei die Funktionsteile des Verschlusses in der Lage dargestellt sind, in welcher Flüssigkeit aus dem Fläschchen in die Dosierkammer überströmen kann, wobei die Schnittführung des Hohlstopfens des Dosier-Verschlusses in Fig. 2 durch die Pfeile 3-3 veranschaulicht ist.

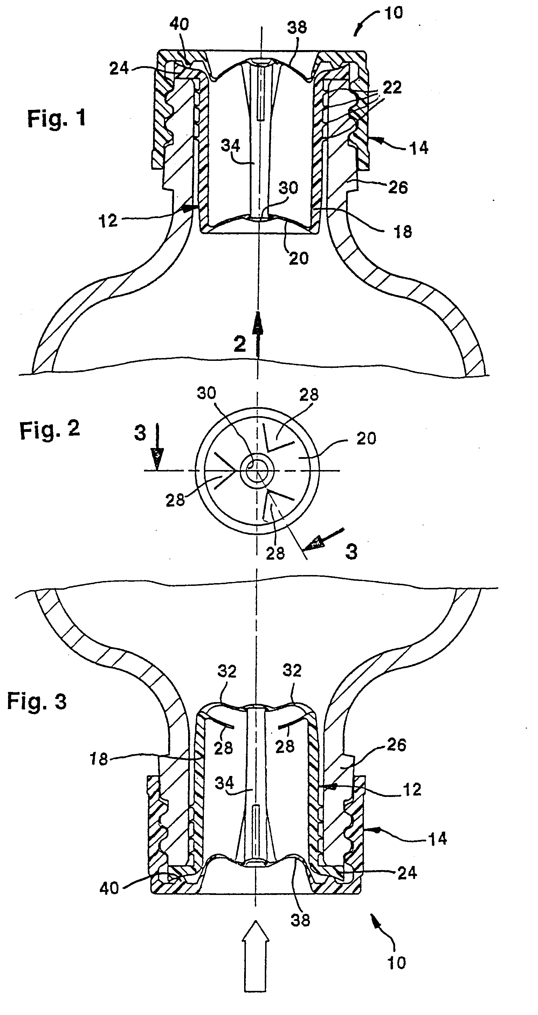

Das in den Figuren 1 und 3 gezeigte Ausführungsbeispiel eines - in seiner Gesamtheit mit 10 bezeichneten erfindungsgemäßen Dosier-Verschlusses setzt sich aus zwei Teilen zusammen, nämlich einem Hohlstopfen 12 und einer im dargestellten Fall als Schraubkappe ausgebildeten Verschlußkappe 14.

Der Hohlstopfen 12 weist eine im wesentlichen zylindrische Umfangswandung 18 auf, die in ihrem in Fig. 1 unten und Fig. 3 oben liegenden behälterinnenseitigen Endbereich durch eine als dünne elastische und kalottenartig hochgewölbte Membran 20 verschlossen ist, in welcher nachstehend in Verbindung mit Fig. 2 noch näher erläuterte zungenartigen Wandabschnitte freigeschnitten oder freigestanzt sind, die sich in der in Fig. 1 dargestellten Ausgangsstellung in die Wölbung der Membran 20 einfügen. Die entlang der freigeschnittenen Begrenzungskanten zwischen den zungenartigen Wandbereichen und der restlichen Membran bestehenden Spalte sind so eng, daß auch bei auf den Kopf gestelltem Verschluß keine Flüssigkeit von außen durch die Membran in den Hohlstopfen 12 eintreten kann. Von dem der Membran 20 gegenüberliegenden oberen offenen Ende der Umfangswandung 18 tritt ein axial nach außen gerichteter Flansch 24 vor, welcher die Einsetztiefe des Hohlstopfens 12 in dem in den Fig. 1 und 3 dargestellten Flaschenhals 26 des mit einer zu dosierenden Flüssigkeit gefüllten Fläschchens begrenzt. Von der Außenseite der Umfangswandung 18 treten eine Reihe von umlaufenden Dichtlamellen 22 vor, die verhindern, daß zwischen der Umfangswandung 18 und dem Flaschenhals 26 Flüssigkeit austreten kann. Der Flansch 24 liegt in der bestimmungsgemäßen Montagestellung des Hohlstopfens auf der ringförmigen Stirnfläche des Flaschenhalses 26 auf und entfaltet - zumindest bei fest aufgeschraubtem Schraubdeckel - eine zusätzliche Dichtwirkung.

In Fig. 2 ist erkennbar, daß in die gewölbte Membran 20 drei in gleichmäßigen Winkelabschnitten zueinander versetzte Zungen oder sektorförmige Wandabschnitte 28 entlang zweier Begrenzungskanten freigeschnitten oder freigestanzt sind, die aber - beim dargestellten Ausführungsbeispiel - in ihrem radial äußeren Bereich noch mit der Membran 20 einstückig verbunden sind. Mittig ist in der Membran 20 eine ihrer Wölbung entgegengerichtete Eintiefung vorgesehen, in welche das freie Ende eines nachstehend noch im Zusammenhang mit der Ausbildung der Verschlußkappe 14 beschriebenen Stößels eingreift. Die freigeschnittenen zungenartigen Wandbereiche 28 der Membran 20 bilden die eigentliche Ventileinrichtung, die in der in Fig. 1 dargestellten Schließstellung das Innere des Fläschchens von der im Innern des Hohlstopfens 12 gebildeten Dosierkammer abschließen. Bei einer Verformung der Membran 20 in Richtung ins Innere des Fläschchens wird die Membran elastisch im Sinne einer Verringerung ihrer Wölbung verformt. Die freigeschnittenen Wandbereiche 28 verformen sich dabei aber nicht in gleicher Weise mit, wodurch sie in dem bisher verschlossenen Membranbereich jeweils einen Durchlaß 32 freigeben, durch den Flüssigkeit in den Hohlstopfen einströmen und gleichzeitig die im Hohlstopfen enthaltene Luft ins Fläschchen entweichen kann, wenn das Fläschchen in der in Fig. 3 gezeigten Weise auf den Kopf gestellt ist.

Die Verformung der Membran 20 zum Zweck der Befüllung der im Innern des Hohlstopfens gebildeten Dosierkammer erfolgt durch den bereits erwähnten Stößel 34, der mittig an der Innenseite des Deckwandbereichs der als Schraubkappe ausgebildeten Verschlußkappe 14 angesetzt ist. Da die Schraubkappe 14 im speziellen Fall - ebenso wie der Hohlstopfen 12 - im Spritzgußverfahren aus thermoplastischem Kunststoff hergestellt ist, ist der Stößel 34 integral in einem zentralen Bereich 38 der Deckwand angespritzt, der durch eine entsprechend dünne und gewölbte Ausbildung - ähnlich der Membran 20 - elastisch verformbar ist, d.h. durch Druck auf seine Außenseite unter elastischer Verformung in den Hohlstopfen hineindrückbar ist. Der in der Eintiefung 30 der Membran 20 abgestützte Stößel 34 verformt dann die Membran in der beschriebenen Weise, wodurch sich die in der Schließstellung durch die zungenartigen Wandabschnitte 28 verschlossenen Durchlässe 32 öffnen. In dem der ringförmigen Stirnfläche des Flaschenhalses 26 gegenüberliegenden Bereich springt von der Innenseite der Deckwand der Verschlußkappe 14 eine ringförmig umlaufende Rippe 40 vor, welche bei fest auf den Flaschenhals aufgeschraubter Schraubkappe 14 auf den radialen Flansch 24 drückt und somit das dichte Anliegen des Flanschs an der Mündung des Flaschenhalses und somit eine zusätzliche Abdichtung zu den im Flaschenhals abdichtenden Ringlamellen 22 bewirkt.

Es ist ersichtlich, daß im Rahmen des Erfindungsgedankens Abwandlungen und Weiterbildungen des beschriebenen Ausführungsbeispiels verwirklichbar sind. So kann beispielsweise die den Hohlstopfen behälterinnenseitig verschließende Membran - dann zweckmäßig mit verstärktem äußerem Rand - gesondert hergestellt und nachträglich im dann beidseitig offenen Hohlstopfen montiert werden. Der als integraler Teil von der Deckwand der Verschlußkappe 14 vortretend beschriebene Stößel 34 kann umgekehrt auch integral an der Membran 20 angespritzt und dann an der Innenseite des verformbaren Deckwandbereichs 38 abgestützt sein. Auch die Zahl und Form der zungenartigen Wandbereiche 28 kann gegenüber dem dargestellten Ausführungsbeispiel abgewandelt sein. Wesentlich ist lediglich, daß die Wandbereiche so ausgebildet und geformt sind, daß sie bei einer Verformung der Membran 20 diese Verformung nicht oder nur teilweise mitmachen, so daß die bis dahin verschlossenen Durchlässe 32 sich öffnen.

## Patentansprüche

1. Dosier-Verschluß (10) für zur Aufnahme von Flüssigkeiten bestimmte Behälter mit einer im Ausguß (26) des die Flüssigkeit aufnehmenden Behälters angeordneten Dosierkammer in Form eines in einem behälterinnenseitig durch eine Stirnwand geschlossenen und ausgußseitig offenen, am Behälterausguß abdichtenden Hohlstopfens (12), in dessen Stirnwand eine federnd in die Schließstellung vorgespannte Ventileinrichtung vorgesehen ist, die zum Übertritt einer vorgegebenen Menge von Flüssigkeit aus dem Behälterinnern in die Dosierkammer geöffnet werden kann, wobei der Behälter und zugleich die offene Stirnseite des Hohlstopfens (12) durch eine abnehmbare Verschlußkappe (14) verschlossen sind, deren dem Durchlaß im Hohlstopfen (12) gegenüberliegender Deckwandbereich (38) elastisch in Richtung ins Hohlstopfeninnere verformbar ausgebildet ist und zwischen der der Dosierkammer zugewandten Innenseite des elastisch verformbaren Deckwandbereichs und der Ventileinrichtung ein die Dosierkammer durchsetzender Stößel (34) angeordnet ist, dessen Länge so bemessen ist, daß er bei einer Verformung des Deckwandbereichs in Richtung des Behälterinnern die Ventileinrichtung aus ihrer Schließstellung in die Öffnungsstellung verstellt,
**dadurch gekennzeichnet**,
daß die Stirnwand des Hohlstopfens (12) als in Richtung zum Behälter-Ausguß durchgewölbte und elastisch in Richtung des Behälterinnern verformbaren Membran (20) ausgebildet ist, an welcher das dem Behälterinnern zugewandte Ende des Stößels (34) angreift und
daß in der Membran (20) wenigstens ein zungenartiger Wandbereich (28) freigeschnitten ist, dessen freigeschnittene Begrenzungskanten in der Schließstellung den angrenzenden Membran-Wandbereichen ohne Bildung eines merklichen Spalts gegenüberstehen, nach einer Verformung der Membran (20) durch den Stößel (34) in die Öffnungsstellung jedoch nicht mit verformt werden und einen Durchlaß in der Membran für den Übertritt von Flüssigkeit aus dem Behälterinnern in die Dosierkammer freigeben.

2. Dosier-Verschluß nach Anspruch 1, dadurch gekennzeichnet, daß der Hohlstopfen (12) einschließlich der die behälterinnenseitige Stirnwand bildenden Membran (20) ein integraler Kunststoff-Spritzgußteil ist.

3. Dosier-Verschluß nach Anspruch 1, dadurch gekennzeichnet, daß die die behälterinnenseitige Stirnwand bildende Membran (20) gesondert hergestellt und nachträglich abdichtend in die behälterinnere Stirnseite der beidseitig offenen hohlzylindrischen Stopfen-Umfangswandung (18) eingesetzt ist.

4. Dosier-Verschluß nach Anspruch 3, dadurch gekennzeichnet, daß die Membran (20) einen verstärkten Randbereich aufweist, welcher in eine im behälterinnenseitigen Endbereich der Stopfen-Umfangswandung in deren Innenseite vorgesehene umlaufende Nut eingerastet ist.

5. Dosier-Verschluß nach Anspruch 4, dadurch gekennzeichnet, daß der Außendurchmesser des verstärkten Randbereichs mit Übermaß gegenüber dem Durchmesser des Nutgrunds der umlaufenden Nut bemessen ist.

6. Dosier-Verschluß nach einem der Ansprüche 1 bis 5, dadurch gekennzeichnet, daß in der die behälterinnenseitige Stirnwand bildenden Membran (20) mehrere zungenartige Wandbereiche (28) freigeschnitten sind.

7. Dosier-Verschluß nach einem der Ansprüche 1 bis 7, dadurch gekennzeichnet, daß der zungenartige Wandbereich bzw. die zungenartigen Wandbereiche (28) in der Membran (20) in einem gesonderten Arbeitsgang freigestanzt ist bzw. sind.

8. Dosier-Verschluß nach einem der Ansprüche 2 bis 7, dadurch gekennzeichnet, daß der zungenartige Wandbereich bzw. die zungenartigen Wandbereiche (28) in der Membran (20) durch entsprechende Ausformung der Spritzgußform bei der Herstellung des Hohlstopfens in der Spritzgußform erzeugt ist bzw. sind.
